# EUROPEAN PATENT APPLICATION

(11) **EP 4 403 224 A2**
(43) Date of publication of application: **24.07.2024**
(21) Application number: 24164847.6
(22) Date of filing: 03.06.2019
(51) Int. Cl.: A61P 35/00

(54) **CHIMERIC ANTIGEN RECEPTOR T CELL THERAPY**

(30) Priority: 01.06.2018 US 201862679755 P; 30.11.2018 US 201862774157 P
(62) Divisional of application: 19810295.6
(71) Applicant: Kite Pharma, Inc., Santa Monica, CA 90404 (US)
(72) Inventor: BOT, Adrian I., 2400 Broadway, 90404 (US); GO, William Y., Thousand Oaks, 91362 (US); JIANG, Yizhou, Santa Monica, 90404 (US); ROSSI, John M., Santa Monica, 90404 (US); XUE, Xiaodong, Santa Monica, 90404 (US)
(74) Representative: Thomann, William John

(57) **Abstract**

The disclosure relates to, in part, methods of CAR-T cancer treatment and methods of predicting clinical outcomes in response to those treatments. The methods comprise the use of baseline Sum of Product Diameters of Index Lesions and baseline number of lines of prior therapy as indicators.

## Description

### Field

The present application is directed to methods of CAR-T cancer treatment and methods of predicting clinical outcomes in response to those treatments.

### Background

Immunotherapy cancer treatments rely on enriched or modified human T cells to target and kill cancer cells in a patient. To increase the ability of T cells to target and kill a particular cancer cell, methods have been developed to engineer T cells to express constructs that direct T cells to a particular target cancer cell. Chimeric antigen receptors (CARs) and engineered T cell receptors (TCRs), which comprise binding domains capable of interacting with a particular tumor antigen, allow T cells to target and kill cancer cells that express the particular tumor antigen. Adaptive cell therapies that target CD19-expressing cancer cells have shown promissing results with superior clinical outcomes. There remains a need to predict clinical outcomes to CAR-T therapies.

### Summary of the Disclosure

The present application relates to methods of CAR-T cancer treatment and methods of predicting clinical outcomes in response to those treatments. The methods of predicting potential clinical outcome comprise the use of baseline Sum of Product Diameters of Index Lesions (SPD) and/or baseline number of lines of prior therapy as indicators.

It is to be understood that the disclosure is not limited in its application to the details set forth in the following embodiments, claims, description and figures. The disclosure is capable of other embodiments and of being practiced or carried out in numerous other ways.

The following are some exemplary embodiments of the disclosure:

Embodiment 1. A method of predicting a response to CD19 CAR-T treatment in a subject having cancer, comprising measuring a baseline SPD in the subject, determining the SPD range, wherein a low SPD range indicates a likelihood of positive response to the CAR-T treatment.

Embodiment 2. The method of embodiment 1, where the SPD can fall within one of 4 ranges, whereby the lower the range in which the SPD falls the higher the likelihood of response to CD19 CAR-T treatment is.

Embodiment 3. The method of embodiment 2, wherein the four ranges comprise the following:
SPD Quartile 1, from about 100 (inclusive) to about 2000 mm² (inclusive), median SPD of about 840;
SPD Quartile 2, from about 2000 (non inclusive) to about 3700 mm² (inclusive), median SPD of about 2820;
SPD Quartile 3, from about 3700 (non inclusive) to about 6700 mm² (inclusive), median SPD of about 5100; and
SPD Quartile 4, from about 6700 (non inclusive) to about 24,000 mm² (inclusive), median SPD of about 9300.

Embodiment 4. The method of any one of embodiments 1 through 3, wherein the subject is subsequently treated with CD19 CAR-T treatment when the baseline SPD value is in the SPD Quartiles 1 through 4.

Embodiment 5. A method of treating cancer in a subject in need thereof, comprising administering a therapeutically effective amount of a CD19 CAR-T treatment to a subject in which the baseline SPD value is in the SPD Quartiles 1 through 4.

Embodiment 6. A method of predicting a likelihood of relapse after CD19 CAR-T treatment in a subject having cancer, comprising determining the number of lines of prior therapy in the subject, determining where the number falls within one of four ranges, whereby the higher the number of lines of prior therapy the higher likelihood of relapse after CD19 CAR-T treatment for the subject is predicted to be.

Embodiment 7. A method of predicting the likelihood of ongoing response to CD19 CAR-T treatment in a subject having cancer, comprising measuring the baseline number of lines of prior therapy in the subject, determining where the number falls within one of four ranges, whereby the lower the range indicates the likelihood of ongoing response to CD19 CAR-T treatment.

Embodiment 8. The method of any one of embodiments 6 and 7, wherein the ranges of number of lines of prior therapy are 1-2; 3; 4; or ≥ 5.

Embodiment 9. The method of any one of embodiments 6 through 8, further comprising subsequently administering CD19 CAR-T treatment to the subject in which the number of lines of prior therapy are 1-2; 3; 4; or ≥ 5.

Embodiment 10. A method of treating cancer in a subject in need thereof, comprising administering a therapeutically effective amount of a CD19 CAR-T treatment to a subject in which the number of lines of prior therapy in the subject is 1-2; 3; 4; or ≥ 5.

Embodiment 11. The method of any one of embodiments 1-10, wherein the cancer is a hematologic cancer or relapsed/refractory diffuse large B cell lymphoma.

Embodiment 12. The method of any one of embodiments 1-11, wherein the CD19 CAR-T treatment comprises treatment with axicabtagene ciloleucel (Yescarta), tisagenlecleucel (Kymriah), JCAR017, JCAR015, JCAR014, Uppsala U. anti-CD19 CAR (NCT02132624), or UCART19.

Embodiment 13. A method of predicting long-term response durability to treatment of cancer with anti-CD19 CAR-T cell treatment in a patient in need thereof, the method comprising assessing progression free survival at 3 months after a single dose of treatment, wherein achievement of complete or partial response at 3 months is predictive of long-term response durability in the patient.

Embodiment 14. The method of embodiment 13, wherein the anti-CD19 CAR-T treatment comprises treatment with axicabtagene ciloleucel (Yescarta), tisagenlecleucel (Kymriah), JCAR017, JCAR015, JCAR014, Uppsala U. anti-CD19 CAR (NCT02132624), or UCART19.

Embodiment 15. The method of embodiment 13 or 14, wherein the cancer is a hematological cancer.

Embodiment 16. The method of embodiment 15, wherein the cancer is relapsed/refractory diffuse large B cell lymphoma.

Embodiment 17. The method of any one of embodiments 13 through 16, wherein long-term response durability comprises a complete or partial response lasting more than 9 months, more than 12 months, more than 18 months, or more than 24 months.

Embodiment 18. The method of any one of embodiments 1 through 17, wherein the subject is ≥ 65 years old.

Embodiment 19. The method of any one of embodiments 1 through 17, wherein the subject is < 65 years old.

Embodiment 20. The method of any one of embodiments 1 through 19, whrein the CD19 CAR-T treatment is administered as first line therapy.

### Brief Description of the Figures

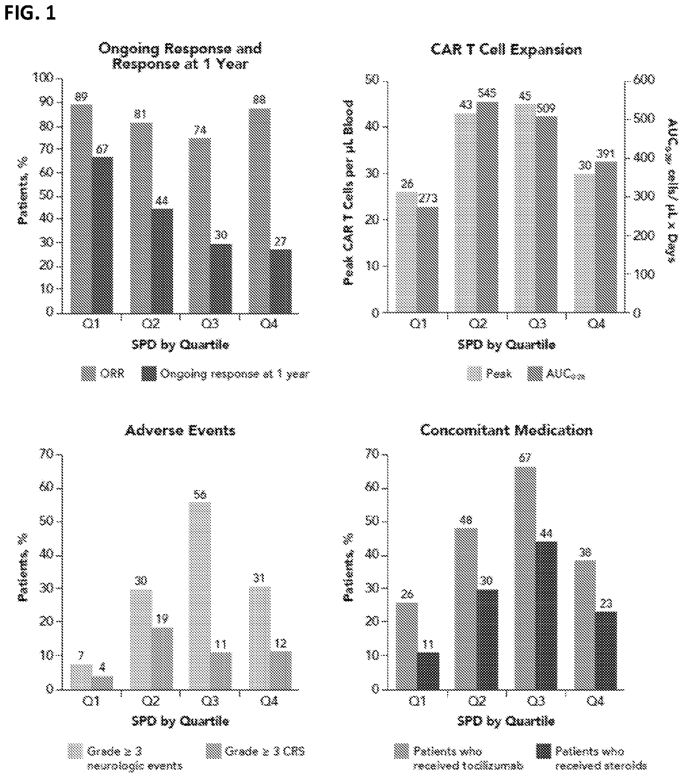

FIG. 1 illustrates the patient outcomes by Quartiles of SPD. AUC₀₋₂₈ (area under the curve from Day 0 to Day 28); CRS (cytokine release syndrome); ORR (objective response rate); Q (quartile).

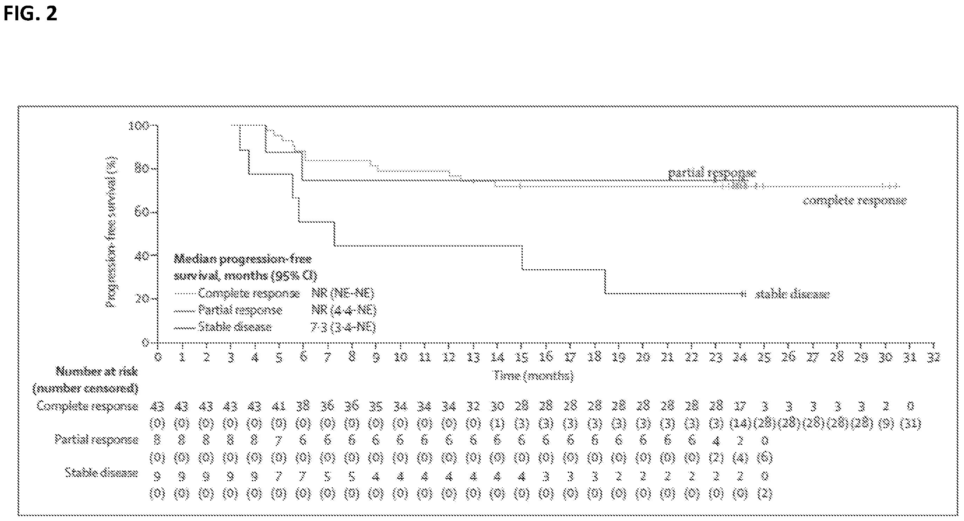

FIG. 2 Post-hoc analysis of investigator-assessed progression-free survival by response status at 3 months after axicabtagene ciloleucel. 60 patients with ongoing complete response, partial response, or stable disease month 3 in phase 2 are shown. The x-axis shows time since infusion of chimeric antigen receptor T cells. Four of eight patients with partial responses and four of nine patients with stable disease at 3 months subsequently converted to complete responses. NR=not reached. NE=not estimable.

### Detailed Description

Unless otherwise defined herein, scientific and technical terms used in connection with the present disclosure shall have the meanings that are commonly understood by those of ordinary skill in the art.

As used in this specification and the appended claims, the singular forms "a," "an" and "the" include plural referents unless the context clearly dictates otherwise . Unless specifically stated or obvious from context, as used herein, the term "or" is understood to be inclusive and covers both "or" and "and". The term "and/or" where used herein is to be taken as specific disclosure of each of the two specified features or components with or without the other.

As described herein, any concentration range, percentage range, ratio range or integer range is to be understood to be inclusive of the value of any integer within the recited range and, when appropriate, fractions thereof (such as one-tenth and one-hundredth of an integer), unless otherwise indicated. Unless specifically stated or evident from context, as used herein, the term "about" refers to a value or composition that is within an acceptable error range for the particular value or composition as determined by one of ordinary skill in the art, which will depend in part on how the value or composition is measured or determined, *i.e.,* the limitations 10 of the measurement system. For example, "about" or "comprising essentially of" can mean within one or more than one standard deviation per the practice in the art. "About" or "comprising essentially of" can mean a range of up to 10% (*i.e.,* ±10%). Thus, "about" can be understood to be within 10%, 9%, 8%, 7%, 6%, 5%, 4%, 3%, 2%, 1%, 0.5%, 0.1%, 0.05%, 0.01%, or 0.001% greater or less than the stated value. For example, about 5 mg can include 15 any amount between 4.5 mg and 5.5 mg. Furthermore, particularly with respect to biological systems or processes, the terms can mean up to an order of magnitude or up to 5-fold of a value. When particular values or compositions are provided in the instant disclosure, unless otherwise stated, the meaning of "about" or "comprising essentially of" should be assumed to be within an acceptable error range for that particular value or composition.

The term "administering" refers to the physical introduction of an agent to a subject, using any of the various methods and delivery systems known to those skilled in the art. Exemplary routes of administration for the formulations disclosed herein include intravenous, intramuscular, subcutaneous, intraperitoneal, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, as well as in vivo electroporation. In some embodiments, the formulation is administered via a non-parenteral route, e.g., orally. Other non-parenteral routes include a topical, epidermal or mucosal route of administration, for example, intranasally, vaginally, rectally, sublingually or topically. Administering can also be performed, for example, once, a plurality of times, and/or over one or more extended periods. In some embodiments, administration is by infusion. For example, an infusion bag of CD19-directed genetically modified autologous T cell immunotherapy comprises a suspension of chimeric antigen receptor (CAR)-positive T cells in approximately 68 mL. The target dose may be between about 1 × 10⁶ and about 2 × 10⁶ CAR-positive viable T cells per kg body weight, with a maximum of 2 × 10⁸ CAR-positive viable T cells. In some embodiments the CD19-directed genetically modified autologous T cell immunotherapy is Axi-cel^{™} (YESCARTA^{®}, axicabtagene ciloleucel)

The term "lymphocyte" as used herein includes natural killer (NK) cells, T cells, or B cells. NK cells are a type of cytotoxic (cell toxic) lymphocyte that represent a major component of the inherent immune system. NK cells reject tumors and cells infected by viruses. It works through the process of apoptosis or programmed cell death. They were termed "natural killers" because they do not require activation in order to kill cells. T-cells play a major role in cell-mediated-immunity (no antibody involvement). Its T-cell receptors (TCR) differentiate themselves from other lymphocyte types. The thymus, a specialized organ of the immune system, is primarily responsible for the T cell's maturation. There are six types of T-cells, namely: Helper T-cells (e.g., CD4+ cells), Cytotoxic T-cells (also known as TC, cytotoxic T lymphocyte, CTL, T-killer cell, cytolytic T cell, CD8+ T-cells or killer T cell), Memory T-cells ((i) stem memory T_{SCM} cells, like naive cells, are CD45RO-, CCR7+, CD45RA+, CD62L+ (L-selectin), CD27+, CD28+ and IL-7R.alpha.+, but they also express large amounts of CD95, IL-2R.beta., CXCR3, and LFA-1, and show numerous functional attributes distinctive of memory cells); (ii) central memory T.sub.CM cells express L-selectin and the CCR7, they secrete IL-2, but not IFN.gamma. or IL-4, and (iii) effector memory TEM cells, however, do not express L-selectin or CCR7 but produce effector cytokines like IFN.gamma. and IL-4), Regulatory T-cells (Tregs, suppressor T cells, or CD4+CD25+ regulatory T cells), Natural Killer T-cells (NKT) and Gamma Delta T-cells. B-cells, on the other hand, play a principal role in humoral immunity (with antibody involvement). It makes antibodies and antigens and performs the role of antigen-presenting cells (APCs) and turns into memory B-cells after activation by antigen interaction. In mammals, immature B-cells are formed in the bone marrow, where its name is derived from.

A "patient" as used herein includes any human who is afflicted with a disease or condition such as cancer (e.g., a lymphoma or a leukemia). The terms "subject" and "patient" may be used interchangeably herein.

The term "immunotherapy" refers to the treatment of a subject afflicted with, or at risk of contracting or suffering a recurrence of, a disease by a method comprising inducing, enhancing, suppressing or otherwise modifying an immune response. Examples of immunotherapy include, but are not limited to, T cell therapies. T cell therapy can include adoptive T cell therapy, tumor-infiltrating lymphocyte (TIL) immunotherapy, autologous cell therapy, engineered autologous cell therapy (eACT.TM.), and allogeneic T cell transplantation. One of skill in the art would recognize that the conditioning methods disclosed herein would enhance the effectiveness of any transplanted T cell therapy. Examples of T cell therapies are described in U.S. Patent Publication Nos. 2014/0154228 and 2002/0006409, U.S. Pat. No. 5,728,388, and International Publication No. WO 2008/081035. Other non-limiting examples can be found in U.S. Pat. No. 9,855,298; U.S. Pat. Appl. Pub. Nos. 20190151361; 20190144515; 20190093101; 20190032011; 20190092818; 20180369283; 20180296601; 20180280437; 20180230224; 20180086846; 20180016620. Exemplary reviews of T cell therapies used in the art include Jafferji MS, Yang JC, Adoptive T-Cell Therapy for Solid Malignancies, Surg Oncol Clin N Am. 2019 Jul;28(3):465-479. doi: 10.1016/j.soc.2019.02.012. Epub 2019 Apr 12; Minutolo NG, Hollander EE, Powell DJ Jr.. The Emergence of Universal Immune Receptor T Cell Therapy for Cancer, Front Oncol. 2019 Mar 26;9:176. doi: 10.3389/fonc.2019.00176. eCollection 2019; Strati P, Neelapu SS., Chimeric Antigen Receptor-Engineered T Cell Therapy in Lymphoma, Curr Oncol Rep. 2019 Mar 27;21(5):38. doi: 10.1007/s 11912-019-0789-z.

The term "autologous" refers to any material derived from the same individual to which it is later to be re-introduced. For example, the autologous cell therapy (ACT) method described herein involves collection of lymphocytes from a patient, which are then engineered to express, e.g., a CAR construct, and then administered back to the same patient.The term "Autologous Cell Therapy," which can be abbreviated as "ACT," also known as adoptive cell transfer, is a process by which a patient's own T cells are collected and subsequently genetically altered to recognize and target one or more antigens expressed on the cell surface of one or more specific tumor cells or malignancies.

By "therapeutically effective" is meant that the use of CD19 CAR-T to treat cancer in a patient results in any demonstrated clinical benefit compared with no therapy (when appropriate) or to a known standard of care. Clinical benefit in a population of patients can be assessed by any method known to one of ordinary skill in the art. In one embodiment, clinical benefit may be assessed based on objective response rate (ORR), duration of response (DOR), progression-free survival (PFS), ongoing response at 1 year, and/or overall survival (OS). Objective Response Rate (ORR) is defined as the proportion of the participants who achieve a complete response (CR) or partial response (PR).

In some embodiments, a complete response indicates therapeutic benefit. In some embodiments, a partial response indicates therapeutic benefit. In some embodiments, stable disease indicates therapeutic benefit. In some embodiments, an increase in overall survival indicates therapeutic benefit. In some embodiments, therapeutically effective may constitute an improvement in time to disease progression and/or an improvement in symptoms or quality of life. In other embodiments, therapeutic benefit may not translate to an increased period of disease control, instead a markedly reduced symptom burden resulting in improved quality of life.

### CHIMERIC ANTIGEN RECEPTOR (CAR)

Chimeric antigen receptors (CARs or CAR-Ts) and the T cell receptors (TCRs) of the disclosure are genetically engineered receptors. These engineered receptors may be readily inserted into and expressed by immune cells, including T cells, in accordance with techniques known in the art. With a CAR, a single receptor can be programmed to both recognize a specific antigen and, when bound to that antigen, activate the immune cell to attack and destroy the cell bearing or expressing that antigen. When these antigens exist on tumor cells, an immune cell that expresses the CAR may target and kill the tumor cell.

An aspect of the present invention is a chimeric antigen receptor (CAR), or a T cell receptor, which comprises (i) an antigen binding molecule, (ii) a costimulatory domain, and (iii) an activating domain. The costimulatory domain may comprise an extracellular domain, a transmembrane domain, and an intracellular domain. In some embodiments, the extracellular domain comprises an hinge, or a truncated hinge domain.

In some embodiments, the antigen-binding molecule is a molecule that comprises the antigen binding parts (e.g., CDRs) of the antibody from which the molecule is derived. An antigen binding molecule may include the antigenic complementarity determining regions (CDRs). Examples of antigen-binding molecules include, but are not limited to, Fab, Fab', F(ab')2, and Fv fragments, dAb, linear antibodies, scFv antibodies, and multispecific antibodies formed from antigen binding molecules. Peptibodies (i.e., Fc fusion molecules comprising peptide binding domains) are another example of suitable antigen binding molecules. In one embodiment, the CD19 CAR construct comprises an anti-CD 19 single-chain FV. A "Single-chain Fv" or "scFv" antibody binding fragment comprises the variably heavy (V_{H}) and variable light (V_{L}) domains of an antibody, where these domains are present in a single polypeptide chain. Generally, the Fv polypeptide further comprises a polypeptide linker between the V_{H} and V_{L} domains, which enables the scFv to form the desired structure for antigen binding. All antibody-related terms used herein take the customary meaning in the art and are well understood by one of ordinary skill in the art.

In some embodiments, the CAR comprises one or more costimulatory domains. In some embodiments, the costimulatory is a signaling region of CD28, OX-40, 4-1BB/CD137, CD2, CD7, CD27, CD30, CD40, programmed death-1 (PD-1), inducible T cell costimulator (ICOS), lymphocyte function-associated antigen-1 (LFA-1 (CDI la/CD18), CD3 gamma, CD3 delta, CD3 epsilon, CD247, CD276 (B7-H3), LIGHT (tumor necrosis factor superfamily member 14; TNFSF14), NKG2C, Ig alpha (CD79a), DAP-10, Fc gamma receptor, MHC class I molecule, TNF receptor proteins, Immunoglobulin-like proteins, cytokine receptors, integrins, signaling lymphocytic activation molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CDI Id, ITGAE, CD103, ITGAL, CDI Ia, LFA-1, ITGAM, CDI Ib, ITGAX, CDI Ic, ITGBI, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, LyI08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, or any combination thereof.

In some embodiments, the intracellular domain comprises a signaling region of 4-1BB/CD137, activating NK cell receptors, B7-H3, BAFFR, BLAME (SLAMF8), BTLA, CD100 (SEMA4D), CD103, CD160 (BY55), CD18, CD19, CD19a, CD2, CD247, CD27, CD276 (B7-H3), CD29, CD3 delta, CD3 epsilon, CD3 gamma, CD30, CD4, CD40, CD49a, CD49D, CD49f, CD69, CD7, CD84, CD8alpha, CD8beta, CD96 (Tactile), CDI Ia, CDI Ib, CDI Ic, CDI Id, CDS, CEACAM1, CRT AM, cytokine receptors, DAP-10, DNAM1 (CD226), Fc gamma receptor, GADS, GITR, HVEM (LIGHTR), IA4, ICAM-1, ICAM-1, Ig alpha (CD79a), IL2R beta, IL2R gamma, IL7R alpha, Immunoglobulin-like proteins, inducible T cell costimulator (ICOS), integrins, ITGA4, ITGA4, ITGA6, ITGAD, ITGAE, ITGAL, ITGAM, ITGAX, ITGB2, ITGB7, ITGBI, KIRDS2, LAT, LFA-1, LFA-1, a ligand that specifically binds with CD83, LIGHT, LIGHT (tumor necrosis factor superfamily member 14; TNFSF14), LTBR, Ly9 (CD229), lymphocyte function-associated antigen-1 (LFA-1 (CDI la/CD18), MHC class I molecule, NKG2C, NKG2D, NKp30, NKp44, NKp46, NKp80 (KLRF1), OX-40, PAG/Cbp, programmed death-1 (PD-1), PSGL1, SELPLG (CD162), signaling lymphocytic activation molecules (SLAM proteins), SLAM (SLAMF1; CD150; IPO-3), SLAMF4 (CD244; 2B4), SLAMF6 (NTB-A; LyI08), SLAMF7, SLP-76, TNF receptor proteins, TNFR2, a Toll ligand receptor, TRANCE/RANKL, VLA1, or VLA-6, or a combination thereof. In some embodiments, the CAR comprises a hinge region between the transmembrane domain and the binding molecule. In some embodiments, the hinge region is of IgG1, IgG2, IgG3, IgG4, IgA, IgD, IgE, IgM, CD28, or CD8 alpha.

In some embodiments, the transmembrane domain is a transmembrane domain of CD28, 4-1BB/CD137, an alpha chain of a T cell receptor, a beta chain of a T cell receptor, CD3 epsilon, CD4, CD5, CD8 alpha, CD9, CD16, CD19, CD22, CD33, CD37, CD45, CD64, CD80, CD86, CD134, CD137, CD154, or a zeta chain of a T cell receptor, or any combination thereof. In some embodiments, the activation domain may be derived from, e.g., any form of CD3-zeta. In some embodiments, the activation domain comes from DAP10, DAP12, or other TCR-type activating signaling molecule.

In one aspect, the present application is directed to CD19 CAR T cell therapy. In one embodiment, the CD19 CAR construct comprises an anti-CD19 scFv domain, an intracellular domain, a transmembrane domain, a costimulatory domain, and an activation domain. In one embodiment, the transmembrane domain is derived from transmembrane domain of CD28, 4-1BB/CD137, CD8 alpha, or any combination thereof. In one embodiment, the costimulatory domain is derived from CD8, CD28 OX40, 4-1BB/CD137, or a combination thereof. In one embodiment, the activation domain is derived from CD3zeta. In one embodiment, the CD19 CAR construct comprises a 4-1BB costimulatory domain. In one embodiment, the CD19 CAR construct comprises a CD28 costimulatory domain. In one embodiment, the CD19 CAR construct comprises and anti-CD19 scFv, hinge/transmembrane and costimulatory domains from CD28, and an activation domain from CD3zeta. In one embodiment, the CAR is that exoressed in axicabtagene ciloleucel. In one embodiment, the CAR is that is expressed in Kymriah^{™}. Additional CD19 directed CARs that may be used with the methods of the disclosure include, but are not limited to, JCAR017, JCAR015, JCAR014, Uppsala U. anti-CD19 CAR (NCT02132624), and UCART19 (Celectis), See Sadelain et al. Nature Rev. Cancer Vol. 3 (2003), Ruella et al., Curr Hematol Malig Rep., Springer, NY (2016) and Sadelain et al. Cancer Discovery (Apr 2013).

### CAR-T CELLS

The T cells of the immunotherapy may be engineered to express any of the CAR described above or others and are referred to as CAR-T cells. CAR-T cells may be engineered to express other molecules and may be of any one of the following exemplary types or others available in the art: first, second, third, fourth, fifth (etc) CAR-T cells; Armored CAR-T cells, Motile CAR-T ceclls, TRUCK T-cells, Switch receptor CAR-T cells; Gene edited CAR T-cels; dual receptor CAR T-cells; suicide CAR T-cells, drug-inducible CAR-T cells, synNotch inducible CAR T-cells; and inhibitory CAR T-cells.

The T cells of the immunotherapy may come from any source known in the art. For example, T cells can be differentiated in vitro from a hematopoietic stem cell population, or T cells can be obtained from a subject. T cells can be obtained from, e.g., peripheral blood mononuclear cells (PBMCs), bone marrow, lymph node tissue, cord blood, thymus tissue, tissue from a site of infection, ascites, pleural effusion, spleen tissue, and tumors. In addition, the T cells can be derived from one or more T cell lines available in the art. T cells can also be obtained from a unit of blood collected from a subject using any number of techniques known to the skilled artisan, such as FICOLL^{™} separation and/or apheresis. Additional methods of isolating T cells for a T cell therapy are disclosed in U.S. Patent Publication No. 2013/0287748. Other non-limiting examples can be found in International Application No. PCT/US2015/014520 (published as WO2015/120096) and in International Application No. PCT/US2016/057983 (published as WO2017/070395), all of which are herein incorporated by reference in their totality for the purposes of describing these methods and in their entirety.

In one embodiment, the T cells are autologous T-cells. In one embodiment, the T cells are autologous stem cells (for autologous stem cell therapy or ASCT). In one embodiment, the T cells are non-autologous T-cells. In some embodiments, the T cells are obtained from a donor subject. In some embodiments, the donor subject is human patient afflicted with a cancer or a tumor. In some embodiments, the donor subject is a human patient not afflicted with a cancer or a tumor. In some embodiments, the donor T cells for use in the T cell therapy are obtained from the patient (e.g., for an autologous T cell therapy). In other embodiments, the donor T cells for use in the T cell therapy are obtained from a subject that is not the patient.

The CD19 CAR-T cells may be prepared by any manufacturing method of preparing T cells for immunotherapy, including, without limitation, those described in International Application No. PCT/US2015/014520 (published as WO2015/120096) and in International Application No. PCT/US2016/057983 (published as WO2017/070395), both of which are herein incorporated by reference in their totality for the purposes of describing these methods; any and all methods used in the preparation of Axicabtagene ciloleucel or Yescarta^{®}; any and all methods used in the preparation of Tisagenlecleucel/Kymriah^{™} ; any and and all methods used in the preparation of "off-the-shelf" T cells for immunotherapy; and any other methods of preparing lymphocytes for administration to humans.

In one embodiment, the T cells may be obtained from, e.g., peripheral blood mononuclear cells, bone marrow, lymph node tissue, cord blood, thymus tissue, tissue from a site of infection, ascites, pleural effusion, spleen tissue, and tumors. In addition, the T cells may be derived from one or more T cell lines available in the art. T cells may also be obtained from a unit of blood collected from a subject using any number of techniques known to the skilled artisan, such as FICOLL^{™} separation and/or apheresis. In some embodiments, the cells collected by apheresis are washed to remove the plasma fraction, and placed in an appropriate buffer or media for subsequent processing. In some embodiments, the cells are washed with PBS. As will be appreciated, a washing step may be used, such as by using a semiautomated flow through centrifuge, e.g., the CobeTM 2991 cell processor, the Baxter CytoMateTM, or the like. In some embodiments, the washed cells are resuspended in one or more biocompatible buffers, or other saline solution with or without buffer. In some embodiments, the undesired components of the apheresis sample are removed. Additional methods of isolating T cells for a T cell therapy are disclosed in U.S. Patent Pub. No. 2013/0287748, which is herein incorporated by references in its entirety.

In some embodiments, T cells are isolated from PBMCs by lysing the red blood cells and depleting the monocytes, e.g., by using centrifugation through a PERCOLL^{™} gradient. In some embodiments, a specific subpopulation of T cells, such as CD4+, CD8+, CD28+, CD45RA+, and CD45RO+ T cells is further isolated by positive or negative selection techniques known in the art. For example, enrichment of a T cell population by negative selection may be accomplished with a combination of antibodies directed to surface markers unique to the negatively selected cells. In some embodiments, cell sorting and/or selection via negative magnetic immunoadherence or flow cytometry that uses a cocktail of monoclonal antibodies directed to cell surface markers present on the cells negatively selected may be used. For example, to enrich for CD4+ cells by negative selection, a monoclonal antibody cocktail typically includes antibodies to CD8, CD11b, CD14, CD16, CD20, and HLA-DR. In some embodiments, flow cytometry and cell sorting are used to isolate cell populations of interest for use in the present disclosure.

In some embodiments, PBMCs are used directly for genetic modification with the immune cells (such as CARs) using methods as described herein. In some embodiments, after isolating the PBMCs, T lymphocytes are further isolated, and both cytotoxic and helper T lymphocytes are sorted into naive, memory, and effector T cell subpopulations either before or after genetic modification and/or expansion.

In some embodiments, CD8+ cells are further sorted into naive, central memory, and effector cells by identifying cell surface antigens that are associated with each of these types of CD8+ cells. In some embodiments, the expression of phenotypic markers of central memory T cells includes CCR7, CD3, CD28, CD45RO, CD62L, and CD127 and are negative for granzyme B. In some embodiments, central memory T cells are CD8+, CD45RO+, and CD62L+ T cells. In some embodiments, effector T cells are negative for CCR7, CD28, CD62L, and CD127 and positive for granzyme Band perforin. In some embodiments, CD4+ T cells are further sorted into subpopulations. For example, CD4+ T helper cells may be sorted into naive, central memory, and effector cells by identifying cell populations that have cell surface antigens.

T cells can be engineered to express, for example, chimeric antigen receptors (CAR) or T cell receptor (TCR). CAR positive (+) T cells are engineered to express an extracellular single chain variable fragment (scFv) with specificity for a particular tumor antigen linked to an intracellular signaling part comprising at least one costimulatory domain and at least one activating domain. The CAR scFv can be designed to target, for example, CD19, which is a transmembrane protein expressed by cells in the B cell lineage, including all normal B cells and B cell malignances, including but not limited to NHL, CLL, and non-T cell ALL. In some embodiments, the CAR is engineered such that the costimulatory domain is expressed as a separate polypeptide chain. Example CART cell therapies and constructs are described in U.S. Patent Publication Nos. 2013/0287748, 2014/0227237, 2014/0099309, and 2014/0050708, and these references are incorporated by reference in their entirety. In some embodiments, the immunotherapy is Autologous Stem Cell Therapy, which can be done according to methods described in the art including those in the EXAMPLES. Any of the components of the CD19 CAR may include modifications and/or mutations that alter the properties of the CAR-T construct and/or cells expressing the construct, such as those that affect tumor recognition, T-cell cytokine production, T-cell proliferation, T-cell activation, T-cell replication, T-cell exhaustion, T-cell survival. Some examples of CD19-targeted CAR constructs are described in US Patent Publication No. 20170281766. In one embodiment, the CD19 CAR is the construct that is expressed in axicabtagene ciloleucel (Yescarta^{®}). In one embodiment, the CD19 CAR is the construct that is expressed in Kymriah^{™}. Additional CD19 directed CAR therapies that may be used with the methods of the disclosure include, but are not limited to, JCAR017, JCAR015, JCAR014, Uppsala U. anti-CD19 CAR (NCT02132624), and UCART19 (Celectis), See Sadelain et al. Nature Rev. Cancer Vol. 3 (2003), Ruella et al., Curr Hematol Malig Rep., Springer, NY (2016) and Sadelain et al. Cancer Discovery (Apr 2013).

In one embodiment, to prepare CD19-directed genetically modified autologous T cell immunotherapy, a patient's own T cells may be harvested and genetically modified ex vivo by retroviral transduction (e.g., gamma retroviral transduction) to express a chimeric antigen receptor (CAR) comprising a murine anti-CD19 single chain variable fragment (scFv) linked to CD28 and CD3-zeta co-stimulatory domains. In some embodiments, the CAR comprises a murine anti-CD19 single chain variable fragment (scFv) linked to 4-1BB and CD3-zeta co-stimulatory domain. The anti-CD19 CAR T cells may be expanded and infused back into the patient, where they may recognize and eliminate CD19-expressing target cells. In some embodiments, the anti-CD19 CAR T cell therapy comprises therapy with YESCARTA^{®} (Axi-cel^{™}; axicabtagene ciloleucel), which is an example of such CD19-directed genetically modified autologous T cell immunotherapy. See Kochenderfer, et al., (J Immunother 2009;32:689 702).

The T cells may be administered at a therapeutically effective amount. For example, a therapeutically effective amount of the T cells may be at least about 10⁴ cells, at least about 10⁵ cells, at least about 10⁶ cells, at least about 10⁷ cells, at least about 10⁸ cells, at least about 10⁹, or at least about 10¹⁰. In another embodiment, the therapeutically effective amount of the T cells is about 10⁴ cells, about 10⁵ cells, about 10⁶ cells, about 10⁷ cells, or about 10⁸ cells. In some embodiments, the therapeutically effective amount of the CAR T cells is about 2 × 10⁶ cells/kg, about 3 × 10⁶ cells/kg, about 4 × 10⁶ cells/kg, about 5 × 10⁶ cells/kg, about 6 × 10⁶ cells/kg, about 7 × 10⁶ cells/kg, about 8 × 10⁶ cells/kg, about 9 × 10⁶ cells/kg, about 1 × 10⁷ cells/kg, about 2 × 10⁷ cells/kg, about 3 × 10⁷ cells/kg, about 4 × 10⁷ cells/kg, about 5 × 10⁷ cells/kg, about 6 × 10⁷ cells/kg, about 7 × 10⁷ cells/kg, about 8 × 10⁷ cells/kg, or about 9 × 10⁷ cells/kg. In some embodiments, the therapeutically effective amount of the CAR-positive viable T cells is between about 1 × 10⁶ and about 2 × 10⁶ CAR-positive viable T cells per kg body weight up to a maximum dose of about 1 × 10⁸ CAR-positive viable T cells. In some embodiments, the therapeutically effective amount of the CAR-positive viable T cells is about 1 × 10⁶ or about 2 × 10⁶ CAR-positive viable T cells per kg body weight up to a maximum dose of about 1 × 10⁸ CAR-positive viable T cells. The same doses without the term about are also within the scope of the disclosure.

In some embodiments, the therapeutically effective amount of the CAR-positive viable T cells is between about 0.4 × 10⁸ and about 2 × 10⁸ CAR-positive viable T cells. In some embodiments, the therapeutically effective amount of the CAR-positive viable T cells is about 0.4 × 10⁸, about 0.5 × 10⁸, about 0.6 × 10⁸, about 0.7 × 10⁸, about 0.8 × 10⁸, about 0.9 × 10⁸, about 1.0 × 10⁸, about 1.1 × 10⁸, about 1.2 × 10⁸, about 1.3 × 10⁸, about 1.4 × 10⁸, about 1.5 × 10⁸, about 1.6 × 10⁸, about 1.7 × 10⁸, about 1.8 × 10⁸, about 1.9 × 10⁸, or about 2.0 × 10⁸ CAR-positive viable T cells. The term "viable T cells" has the meaning customary in the art.

In some embodiments, the T cell composition comprises a pharmaceutically acceptable carrier, diluent, solubilizer, emulsifier, preservative and/or adjuvant. In some embodiments, the composition comprises an excipient. The components of T cell compositions are easily determined by one of ordinary skill in the art.

### METHODS OF PREDICTING RESPONSE TO CD19 CAR-T TREATMENT

Data provided herein demonstrates that (SPD) baseline Sum of Product Diameters of Index Lesions in a subject correlates with the degree of positive response to CD19 CAR-T treatment. Accordingly, in one embodiment, the disclosure provides a method of predicting a response to CD19 CAR-T treatment in a subject having cancer, comprising measuring a baseline Sum of Product Diameters of Index Lesions (SPD) in the subject, determining the SPD range, wherein a low SPD range indicates a likelihood of positive response to the CAR-T treatment.

In one embodiment, the SPD of certain value may be useful to indicate a likelihood of response to CAR-T treatment. In one embodiment, the SPD may fall within one of at least four ranges or quantiles, whereby the lower the range in which the SPD falls would correlate to the higher likelihood of response to CD19 CAR-T treatment. In one embodiment, the four ranges comprise the following: SPD Quartile 1, from about 100 (inclusive) to about 2000 mm² (inclusive), median SPD of about 840; SPD Quartile 2, from about 2000 (non inclusive) to about 3700 mm² (inclusive), median SPD of about 2820; SPD Quartile 3, from about 3700 (non inclusive) to about 6700 mm² (inclusive), median SPD of about 5100; and SPD Quartile 4, from about 6700 (non inclusive) to about 24,000 mm² (inclusive), median SPD of about 9300. In one embodiment, the baseline SPD is measured by Cheson 2007 criteria (Neelapu SS and Locke FL, et al. Blood. 2016;128:LBA-6) by investigator assessment.

The clinical response may be measured by any parameter known in the cancer treatment art, including CR or complete response; ORR or objective response rate; PR or partial response; and ORR orongoing response rate. In one embodiment, the response is measured as the ongoing response rate assessed at at least 1 year of CD19 CAR-T treatment.

In one embodiment, the subject that is predicted to respond to CD-19 CAR-T treatment according to the present application is subsequently treated with a CD19 CAR-T treatment, wherein the subject received at least one prior treatment and not CAR-T treatment, wherein the SPD value of the subject is any of the above listed SPD quartiles. In one embodiment, the subject is further or subsequently treated with a CD19 CAR-T treatment when subject falls within SPD Quartile 1. In one embodiment, the subject is further or subsequently treated with a CD19 CAR-T treatment when subject falls within SPD Quartile 2. In one embodiment, the response is ongoing response at 1 year of treatment treatment, the cancer is relapsed/refractory large diffuse B cell lymphoma, and the treatment is Yescarta ASCT as described in the ZUMA study (see EXAMPLES).

### EXEMPLARY CANCERS

A "cancer" refers to a broad group of various diseases characterized by the uncontrolled growth of abnormal cells in the body. Unregulated cell division and growth results in the formation of malignant tumors that invade neighboring tissues and may also metastasize to distant parts of the body through the lymphatic system or bloodstream. A "cancer" or "cancer tissue" may include a tumor. Examples of cancers that may be treated by the methods of the present disclosure include and are not limited to, cancers of the immune system including lymphoma, leukemia, myeloma, and other leukocyte malignancies. In some embodiments, the methods of the present disclosure may be used to reduce the tumor size of a tumor derived from, for example, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, multiple myeloma, Hodgkin's Disease, non-Hodgkin's lymphoma (NHL), primary mediastinal large B cell lymphoma (PMBC), diffuse large B cell lymphoma (DLBCL), follicular lymphoma (FL), transformed follicular lymphoma, splenic marginal zone lymphoma (SMZL), cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemia, acute myeloid leukemia, chronic myeloid leukemia, acute lymphoblastic leukemia (ALL) (including non T cell ALL), chronic lymphocytic leukemia (CLL), solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, other B cell malignancies, and combinations of said cancers. In one particular embodiment, the cancer is multiple myeloma. In one embodiment, the methods of the present application is suitable to cancer that are be responsive to chemo- or radiation therapy, cancer may be resistant to chemo-or radiation therapy, or cancer that is refractory or relapsed. In one embodiment, a refractory cancer refers to a cancer that is not amendable to surgical intervention and the cancer is either initially unresponsive to chemo- or radiation therapy or the cancer becomes unresponsive over time.

In another embodiment, refractory (resistant) disease is suggested by a less than 50 percent decrease in lesion size with treatment in the absence of new lesion development. In contrast progressive disease usually manifests as the appearance of any new lesion, a 50 percent increase in the longest diameter of a previously identified lesion or new/recurrent involvement in the bone marrow. In some embodiments, relapsed disease reflects the appearance of any new lesion after attainment of an initial complete remission. In some embodiments, refractory or progressive disease is identified during the post-treatment response evaluation. Relapses are usually symptomatic and rarely identified solely on the basis of routine imaging. Progressive or relapse may present with systemic B symptoms (i.e. fever, night sweats, weight loss), cytopenias, the development of an extranodal mass, or as the symptomatic or asymptomatic enlargement of the lymph nodes, liver or spleen.

In some embodiments, the cancer is acute lymphoblastic leukemia (ALL) (including non T cell ALL), acute myeloid leukemia (AML), B cell prolymphocytic leukemia, B-cell acute lymphoid leukemia (BALL), blastic plasmacytoid dendritic cell neoplasm, Burkitt's lymphoma, chronic lymphocytic leukemia (CLL), chronic myelogenous leukemia (CML), chronic myeloid leukemia, chronic or acute leukemia, diffuse large B cell lymphoma (DLBCL), follicular lymphoma (FL), hairy cell leukemia, Hodgkin's Disease, malignant lymphoproliferative conditions, MALT lymphoma, mantle cell lymphoma, Marginal zone lymphoma, monoclonal gammapathy of undetermined significance (MGUS), multiple myeloma, myelodysplasia and myelodysplastic syndrome, non-Hodgkin's lymphoma (NHL), plasma cell proliferative disorder (including asymptomatic myeloma (smoldering multiple myeloma or indolent myeloma), plasmablastic lymphoma, plasmacytoid dendritic cell neoplasm, plasmacytomas (including plasma cell dyscrasia; solitary myeloma; solitary plasmacytoma; extramedullary plasmacytoma; and multiple plasmacytoma), POEMS syndrome (also known as Crow-Fukase syndrome; Takatsuki disease; and PEP syndrome), primary mediastinal large B cell lymphoma (PMBC), small cell- or a large cell-follicular lymphoma, splenic marginal zone lymphoma (SMZL), systemic amyloid light chain amyloidosis, T-cell acute lymphoid leukemia (TALL), T-cell lymphoma, transformed follicular lymphoma, or Waldenstrom macroglobulinemia, or a combination thereof.

In some embodiments, the cancer is an hematologic cancer. In one embodiment, the cancer is DLBCL or diffuse large B cell lymphoma; PMBCL orprimary mediastinal B cell lymphoma; or TFL or transformed follicular lymphoma. In one embodiment, the cancer is relapsed or refractory large diffuse B cell lymphoma (DLBCL). In one aspect, the invention provides a method of treating relapsed or refractory diffuse large B-cell lymphoma (DLBCL) not otherwise specified, primary mediastinal large B-cell lymphoma, high grade B-cell lymphoma, or DLBCL arising from follicular lymphoma after two or more lines of systemic therapy in a patient comprising: administering to the patient in need thereof a CD19-directed genetically modified autologous T cell suspension by intravenous infusion at a dose between about 1 × 10⁶ and about 2 × 10⁶ CAR-positive viable T cells per kg body weight up to a maximum dose of about 1 × 10⁸ CAR-positive viable T cells.

### METHODS OF TREATING A SUB-POPULATION OF CANCER SUBJECTS

In some embodiments, the methods of the disclosure may be used to treat a cancer in a subject, reduce the size of a tumor, kill tumor cells, prevent tumor cell proliferation, prevent growth of a tumor, eliminate a tumor from a patient, prevent relapse of a tumor, prevent tumor metastasis, induce remission in a patient, or any combination thereof. In certain embodiments, the methods induce a complete response. In other embodiments, the methods induce a partial response

Data provided herein indicates that SPD or the baseline Sum of Product Diameters of Index Lesions in a subject correlates with the degree of positive response to CD19 CAR-T treatment. Accordingly, in another embodiment, the disclosure provides a method of treating cancer in a subject in need thereof, comprising administering a therapeutically effective amount of CD19 CAR-T treatment to a subject in which the baseline SPD value is SPD Quartile 1. In one embodiment, the disclosure provides a method of treating cancer in a subject in need thereof, comprising administering a therapeutically effective amount of CD19 CAR-T treatment to a subject in which the baseline SPD value is SPD Quartile 2. The cancer may be any one of the above listed cancers. The CD19 CAR-T treatment may be any one of the above listed CD19 CAR-T treatments. In one embodiment, baseline SPD is measured by any of the methods described above.

The higher the SPD quartile, the lower the response. Response and therapeutic benefit are correlated. In some embodiments, a complete response indicates therapeutic benefit. In some embodiments, a partial response indicates therapeutic benefit. In some embodiments, stable disease indicates therapeutic benefit. In some embodiments, an increase in overall survival indicates therapeutic benefit. In some embodiments, therapeutically effective may constitute an improvement in time to disease progression and/or an improvement in symptoms or quality of life. In other embodiments, therapeutic benefit may not translate to an increased period of disease control, instead a markedly reduced symptom burden resulting in improved quality of life.

In another embodiment, the disclosure provides a method of treating cancer in a subject in need thereof, comprising administering a therapeutically effective amount of CD19 CAR-T treatment to a subject in which the number of lines of prior therapy are 1-2; 3; 4; or ≥ 5. In one embodiment, the disclosure provides a method of treating cancer in a subject in need thereof, comprising administering a therapeutically effective amount of CD19 CAR-T treatment to a subject in which the the number of lines of prior therapy are 1-2. The cancer may be any one of the above listed cancers. The CD19 CAR-T treatment may be any one of the above listed CD19 CAR-T treatments. In some embodiments, the CD19 CAR-T treatment is used as first line of treatment.

The lines of prior therapy may be any prior anti-cancer therapy, including, but not limited to Bruton Tyrosine Kinase inhibitor (BTKi), check-point inhibitors (e.g., anti-PD1 antibodies, pembrolizumab (Keytruda), Cemiplimab (Libtayo), nivolumab (Opdivo); anti-PD-L1 antibodies, Atezolizumab (Tecentriq), Avelumab (Bavencio), Durvalumab (Imfinzi); anti-CTLA-4 antibodies, Ipilimumab (Yervoy)), anti-CD19 antibodies (e.g. blinatumomab), anti-CD52 antibodies (e.g. alentuzumab); allogeneic stem cell transplantation, anti-CD20 antibodies (e.g., rituximab), systemic chemotherapy with or without rituximab, rituximab, anthracycline, xxxxxxxxx . The prior therapies may also be used in combination with the CD19 CAR T therapies of the disclosure. In one embodiment, the eligible patients may have refractory disease to the most recent therapy or relapse within 1 year after autologous hematopoietic stem cell transplantation (HSCT/ASCT). In some embodiments, the refractory disease is refractory large B cell lymphoma (diffuse large B cell lymphoma, primary mediastinal B cell lymphoma, transformed follicular lymphoma) or relapsed/refractory CLL. In another embodiment, eligible patients have relapsed or refractory LBCL with ≥ 2 prior systemic therapies. In another embodiment, the patients have acute lymphoblastic leukemia (ALL).

In one embodiment, the disclosure provides that T cell immunotherapy with anti-CD19 CAR-T may induce high rates of durable response with a manageable safety profile for patients ≥ 65 years old. In another embodiment, the disclosure provides that T cell immunotherapy with anti-CD19 CAR-T may induce high rates of durable response with a manageable safety profile for patients < 65 years old. In another embodiment, the disclosure provides that anti-CD19 CAR-T treatment with axi-cell/Yescarta may induce high rates of durable response with a manageable safety profile for patients ≥ 65 years old. In another embodiment, the disclosure provides that anti-CD19 CAR-T treatment with axi-cell/Yescarta may induce high rates of durable response with a manageable safety profile for patients < 65 years old.

In one embodiment, the disclosure provides a method of treating cancer in a subject in need thereof, the method comprising administering a therapeutically effective CD19 CAR T therapy to the subject, wherein the subject is ≥ 65 years old. In one embodiment, the disclosure provides a method of treating cancer in a subject in need thereof, the method comprising administering a therapeutically effective CD19 CAR T therapy to the subject, wherein the subject is < 65 years old. In one embodiment, a "CD19 CAR T therapy" refers to the number of CAR T cells that is administered. In one embodiment, a "CD19 CAR T therapy" refers to the dosage regimen, which comprises the amount of CAR T cells and the frequency and timing of administration, with or without preconditioning.

Amounts of CAR T cells, dosage regimens, methods of administration, subjects, cancers, that fall within the scope of these methods are described elsewhere in this disclosure, alone or in combination with another chemotherapeutic agent, with or without preconditioning, and to any of the patients described elsewhere in the specification.

In one embodiment, the cancer is any of the cancers described above. In one embodiment, the CD19 CAR-T treatment is any of the of CD19 CAR-T treatments described above. In one embodiment, the CD19 CAR-T treatment comprises Yescarta treatment. In one embodiment, the CD19 CAR-T treatment comprises the treatment or protocol described in the ZUMA-1 study (see EXAMPLES). In one embodiment, the response is ongoing response at 1 year of treatment, the cancer is relapsed/refractory large diffuse B cell lymphoma, and the treatment is Yescarta^{®} ASCT as described in the ZUMA study (see EXAMPLES).

In one embodiment, the CD19 CAR-T treatment is any of the of CD19 CAR-T treatments described above. In one embodiment, the CD19 CAR-T treatment comprises Yescarta treatment. In one embodiment, the CD19 CAR-T treatment comprises the treatment or protocol described in the ZUMA-1 study (see EXAMPLES). In one embodiment, the response is ongoing response at 1 year of treatment, the cancer is relapsed/refractory large diffuse B cell lymphoma, and the treatment is Yescarta^{®} ASCT as described in the ZUMA-1 study (see EXAMPLES).

In one embodiment, the cancer is refractory DLBCL diffuse large B cell lymphoma (DLBCL), primary mediastinal B cell lymphoma (PMBCL), or transformed follicular lymphoma (TFL), and, optionally also with (i) no response to last chemotherapy or relapse ≤ 12 mo post-ASCT and (ii) prior anti-CD20 monoclonal antibody and anthracycline and the subjects are administered with a conditioning regimen of cyclophosphamide 500 mg/m² and fludarabine 30 mg/m² for 3 days, followed by a dose of Axi-cel of 2 × 10⁶ CAR+ cells/kg.

In some embodiments, the methods further comprise administering a chemotherapeutic agent or an additional therapeutic agent. In some embodiments, the chemotherapeutic agent selected is a lymphodepleting (preconditioning) chemotherapeutic. Beneficial preconditioning treatment regimens, along with correlative beneficial biomarkers. those described in U.S. Provisional Patent Applications 62/262,143 and 62/167,750 which are hereby incorporated by reference in their entirety herein. These describe, e.g., methods of conditioning a patient in need of a T cell therapy comprising administering to the patient specified beneficial doses of cyclophosphamide (between 200 mg/m²/day and 2000 mg/m²/day) and specified doses of fludarabine (between 20 mg/m²/day and 900 mg/m²/day). One such dose regimen involves treating a patient comprising administering daily to the patient about 500 mg/m²/day of cyclophosphamide and about 60 mg/m²/day of fludarabine for three days prior to administration of a therapeutically effective amount of engineered T cells to the patient. Other examples of preconditioning dose regimens may be found, for example, in U.S. Patent No. 9,855,298.

A variety of additional therapeutic agents may be used in conjunction with the compositions described herein. For example, potentially useful additional therapeutic agents include PD-1 inhibitors such as nivolumab (OPDIVO^{®}), pembrolizumab (KEYTRUDA^{®}), pembrolizumab, pidilizumab (CureTech), and atezolizumab (Roche). Additional therapeutic agents suitable for use in combination with the compositions and methods disclosed herein include, but are not limited to, ibrutinib (IMBRUVICA^{®}), ofatumumab (ARZERRA^{®}), rituximab (RITUXAN^{®}), bevacizumab (AVASTIN^{®}), trastuzumab (HERCEPTIN^{®}), trastuzumab emtansine (KADCYLA^{®}), imatinib (GLEEVEC^{®}), cetuximab (ERBITUX^{®}), panitumumab (VECTIBIX^{®}), catumaxomab, ibritumomab, ofatumumab, tositumomab, brentuximab, alemtuzumab, gemtuzumab, erlotinib, gefitinib, vandetanib, afatinib, lapatinib, neratinib, axitinib, masitinib, pazopanib, sunitinib, sorafenib, toceranib, lestaurtinib, axitinib, cediranib, lenvatinib, nintedanib, pazopanib, regorafenib, semaxanib, sorafenib, sunitinib, tivozanib, toceranib, vandetanib, entrectinib, cabozantinib, imatinib, dasatinib, nilotinib, ponatinib, radotinib, bosutinib, lestaurtinib, ruxolitinib, pacritinib, cobimetinib, selumetinib, trametinib, binimetinib, alectinib, ceritinib, crizotinib, aflibercept,adipotide, denileukin diftitox, mTOR inhibitors such as Everolimus and Temsirolimus, hedgehog inhibitors such as sonidegib and vismodegib, CDK inhibitors such as CDK inhibitor (palbociclib), Bruton Tyrosine Kinase inhibitor (BTKi), check-point inhibitors (e.g., anti-PD1 antibodies, pembrolizumab (Keytruda), Cemiplimab (Libtayo), nivolumab (Opdivo); anti-PD-L1 antibodies, Atezolizumab (Tecentriq), Avelumab (Bavencio), Durvalumab (Imfinzi); anti-CTLA-4 antibodies, Ipilimumab (Yervoy)), anti-CD19 antibodies (e.g. blinatumomab), anti-CD52 antibodies (e.g. alentuzumab); allogeneic stem cell transplantation, anti-CD20 antibodies (e.g., rituximab), systemic chemotherapy with or without rituximab, rituximab, anthracycline, cytokines, other anti-inflammatory agents and the like . The prior therapies may also be used in combination with the CD19 CAR T therapies of the disclosure.

Other examples of chemotherapeutic agents that may be used in combination with the anti-CD19 treatments of the disclosure include alkylating agents such as thiotepa and cyclophosphamide (CYTOXAN^{™}); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine resume; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK^{®}; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL^{™}, Bristol-Myers Squibb) and doxetaxel (TAXOTERE^{®}, Rhone-Poulenc Rorer); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS2000; difluoromethylomithine (DMFO); retinoic acid derivatives such as Targretin^{™} (bexarotene), Panretin^{™}, (alitretinoin); ONTAK^{™} (denileukin diftitox); esperamicins; capecitabine; and pharmaceutically acceptable salts, acids or derivatives of any of the above. In some embodiments, compositions comprising CAR- and/or TCR-expressing immune effector cells disclosed herein may be administered in conjunction with an anti-hormonal agent that acts to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and antiandrogens such as flutamide, nilutamide, bicalutamide, leuprolide, and goserelin; and pharmaceutically acceptable salts, acids or derivatives of any of the above. Combinations of chemotherapeutic agents are also administered where appropriate, including, but not limited to CHOP, i.e., Cyclophosphamide (Cytoxan^{®}), Doxorubicin (hydroxydoxorubicin), Vincristine (Oncovin^{®}), and Prednisone.

In some embodiments, the antigen binding molecule (e.g., antibodies listed above), transduced (or otherwise engineered) cells (such as CARs), and the chemotherapeutic agent are administered each in an amount effective to treat the disease or condition in the subject, alone or in combination with other agents and treatments described herein.

### METHODS OF PREDICTING A LIKELIHOOD OF RELAPSE

Data provided herein indicates that the number of lines of prior therapy in a subject correlates with the degree or likelihood of relapse after CD19 CAR-T treatment. Accordingly, in one embodiment, the disclosure provides a method of predicting a likelihood of relapse after CD19 CAR-T treatment in a subject having cancer, comprising determining the number of lines of prior therapy in the subject, determining where the number falls within one of at least four ranges, whereby the higher the number of lines of prior therapy the higher likelihood of relapse after treatment for the subject is predicted to be.

In one embodiment, the four ranges of number of lines of prior therapy are 1-2; 3; 4; and ≥ 5. In one embodiment, the lines of prior therapy comprise any one or more of prior anti-CD20 monoclonal antibody, anthracyclinethe appropriate standard of care, and combinations of the same. In one embodiment, the predicting method further comprising administering CD19 CAR-T treatment to the subject with a number of prior lines of treatment of any of the ranges described above. In one embodiment, the treatment is administed to subjects having been subject to 1-2 lines of prior therapy. In one embodiment, the degree of relapse is measured at ≤ 12 mo post-treatment, the cancer is relapsed/refractory large diffuse B cell lymphoma, and the treatment is Yescarta ASCT as described in the ZUMA study (see EXAMPLES).

### METHODS OF PREDICTING THE LIKELIHOOD OF RESPONSE TO CAR-T TREATMENT IN A SUBJECT HAVING CANCER

Data provided herein also indicates that the number of lines of prior therapy in a subject correlates with the degree of positive response to CD19 CAR-T treatment. Accordingly, the disclosure also provides methods of predicting the likelihood of response to CD19 CAR-T treatment in a subject having cancer, comprising measuring the baseline number of lines of prior therapy in the subject, determining where the number falls within one of 4 ranges, whereby the lower the range in which the subject falls the higher the likelihood of ongoing response to CD19 CAR-T treatment is.

In one embodiment, the four ranges of number of lines of prior therapy are 1-2; 3; 4; and ≥ 5. In one embodiment, the lines of prior therapy comprise any one or more of prior anti-CD20 monoclonal antibody, anthracycline, cyclophosphamide, fludarabine, the appropriate standard of care, and combinations of the same.

In one embodiment, the cancer is any of the above listed. In one embodiment, the CD19 CAR-T treatment comprises any of those disclosed above.

In one embodiment, the predicting method further comprising administering CD19 CAR-T treatment to the subject with a number of prior lines of treatment of any of the ranges described above. In one embodimen, the treatment is administed to subjects having been subject to 1-2 lines of prior therapy. In one embodiment, the response is ongoing response at 1 year of treatment, the cancer is relapsed/refractory large diffuse B cell lymphoma, and the treatment is Yescarta ASCT as described in the ZUMA study (see EXAMPLES).

The clinical response may be measured by any parameter known in the cancer treatment art, including CR or complete response; ORR or objective response rate; PR or partial response; and ORR or ongoing response rate. In one embodiment, the response is measured as the ongoing response that is assessed at 1 year of CD19 CAR-T treatment.

The following non-limiting examples and data illustrate various aspects and features relating to the methods and uses of the cells and therapies of the present disclosure. In some embodiments, the present methods and uses of compounds provide results and data that are surprising, unexpected and contrary thereto. While the utility of the methods of the disclosure is illustrated through the use of several cells or compounds that can be used therewith, it will be understood by those skilled in the art that comparable results are obtainable with various other cells or compounds, as are commensurate with the scope of this disclosure.

In the context of these examples, ASCT, means autologous stem cell transplant; DLBCL, diffuse large B cell lymphoma; ECOG, Eastern Cooperative Oncology Group performance status; IPI, International Prognostic Index; PD, progressive disease; PMBCL, primary mediastinal B cell lymphoma; SPD, sum of product diameters; and TFL transformed follicular lymphoma.

### EXAMPLES

### Example 1

### Outcomes by Prior Lines of Therapy in Pivotal Phase 2 Study of Axicabtagene Ciloleucel in Patients With Refractory Large B Cell Lymphoma.

Axicabtagene ciloleucel (axi-cel) is an autologous chimeric antigen receptor (CAR) T cell therapy, which recognizes and eliminates CD19-expressing cells. In the pivotal Phase 1/2 multicenter trial (ZUMA-1), 108 patients with refractory large B cell lymphoma were treated (median follow-up, 15.4 months) and showed objective response rate of 82%, with 58% complete responses (CRs); ongoing responses in 42%, including 40% with CRs; 13% Grade ≥ 3 cytokine release syndrome (CRS); 28% Grade ≥ 3 neurologic events.

In Phase 1, subjects (n=7) had Refractory DLBCL diffuse large B cell lymphoma (DLBCL), primary mediastinal B cell lymphoma (PMBCL), or transformed follicular lymphoma (TFL). Phase 2 consisted of two cohorts. Subjects in cohort 1 (n=77) had refractory DLBCL. Subjects in cohort 2 (n=24) had refractory PMBCL/TFL. Additional eligibility criteria included (i) no response to last chemotherapy or relapse ≤ 12 mo post-ASCT and (ii) prior anti-CD20 monoclonal antibody and anthracycline. All subjects were administered with a conditioning regimen of cyclophosphamide 500 mg/m² and fludarabine 30 mg/m² for 3 days. All patients received a dose of Axi-cel of 2 × 10⁶ CAR+ cells/kg. 99% of the subjects enrolled were successfully manufactured and 91% of the enroled were dosed.

The treatment involved several steps in the following order: screening, leukapheresis, and conditioning chemotherapy wee followed by Axi-cell infusion. Manufacturing took place betwen leukapheris and conditioning chemotherapy. Axi-Cel infusion occurred at Day 0. After Day 7, during the follow-up period there was posttreatment assessment and long-term follow-up. The first tumor assessment took place at Day 28. Bridging chemotherapy was not allowed per study protocol.

The following parameters were used for the assessments and statistical analyses: safety and efficacy outcomes were assessed by number of prior lines of therapy: 1- 2, 3, 4, or ≥ 5 prior lines of therapy; autologous stem cell transplant was considered a prior line of therapy; safety and efficacy outcomes were assessed by quartiles of tumor burden; tumor burden was estimated as the sum of product diameters of index lesions (SPD) per Cheson 2007 criteria (Neelapu SS and Locke FL, et al. Blood. 2016;128:LBA-6) by investigator assessment; and/or Index lesion SPD may not represent the totality of a patient's disease.

Results showed that patients with more lines of prior therapy were more likely to have relapsed after ASCT as well as have higher International Prognostic Index scores and disease stage. This is consistent with higher SPD (Table 1).

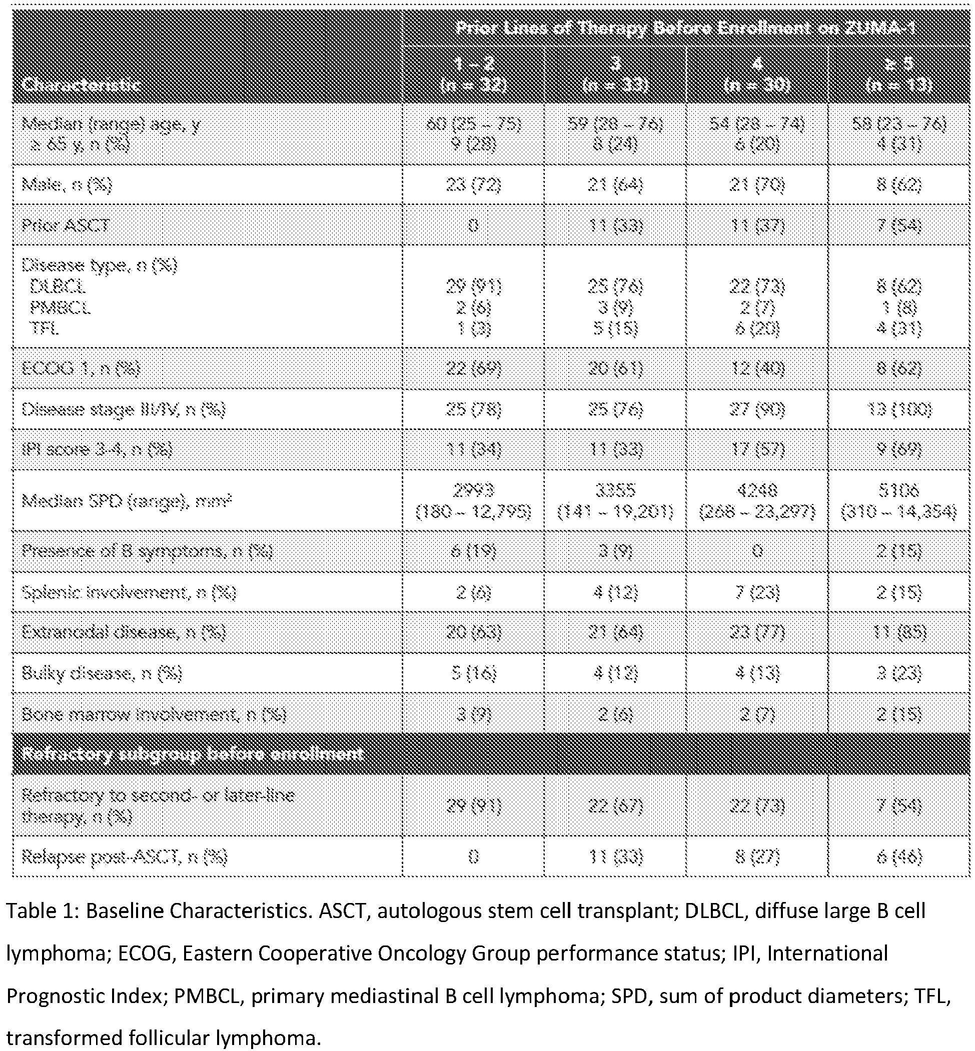

Also, product characteristics were similar across prior lines of therapy (see Table 2 and Table 3). The lines of therapy were counted before enrollment on ZUMA-1.

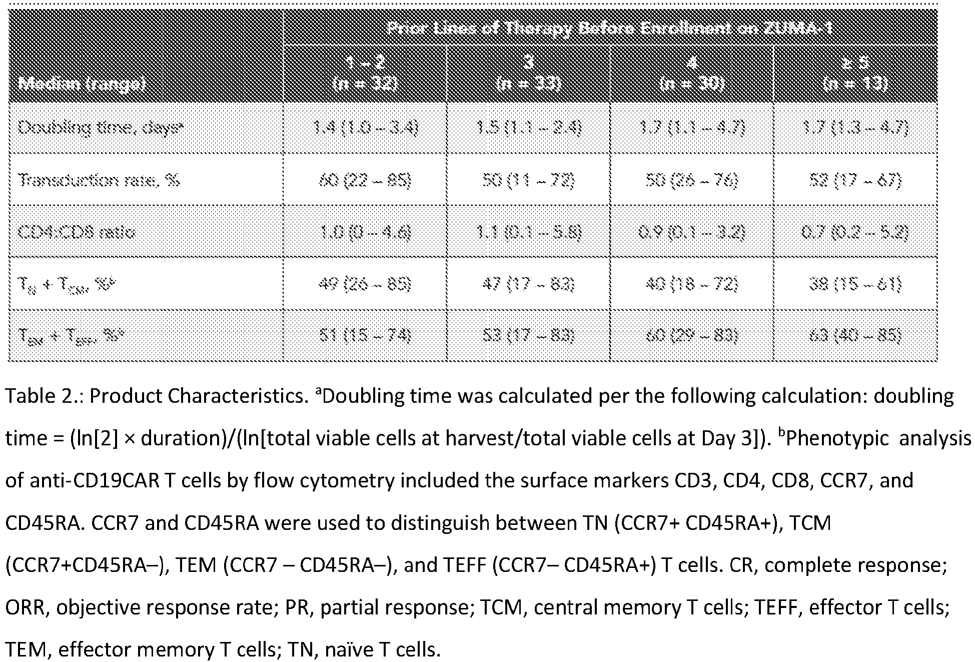

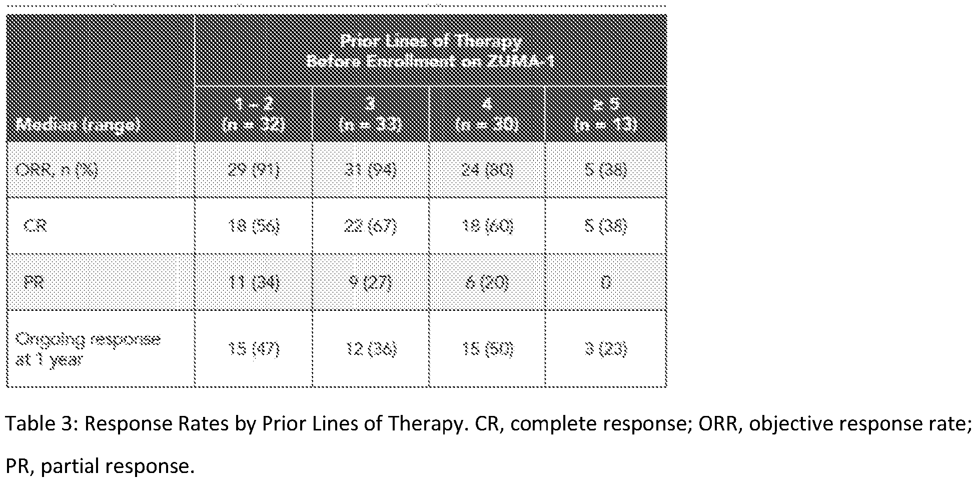

Further, the rates of Grade ≥ 3 CRS were similar across prior lines of therapy (data not shown). There was a trend for increased rates of Grade ≥ 3 serious adverse events and neurologic events for patients with ≥ 5 prior lines of therapy.

Table 4 shows the CAR T cell Expansion by prior lines of therapy. The number was calculated before enrollment on ZUMA-1.

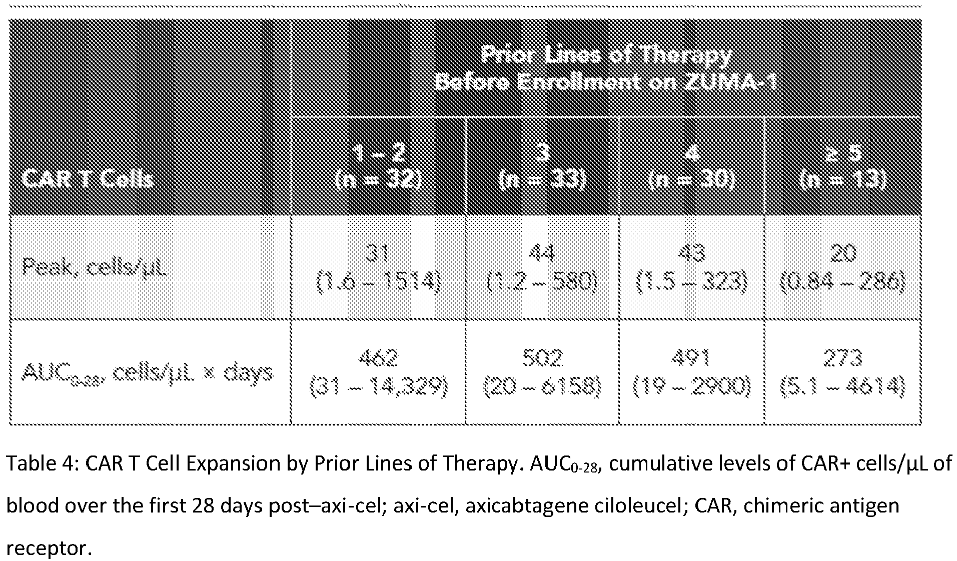

Baseline characteristics suggest that patients with more lines of prior therapy had higher IPI and disease stage. In addition, an evaluation of outcomes by indexed SPD was performed. The results shown in Table 5 suggest that patients in the lowest quartile of SPD (median 840 mm²) had substantial disease.

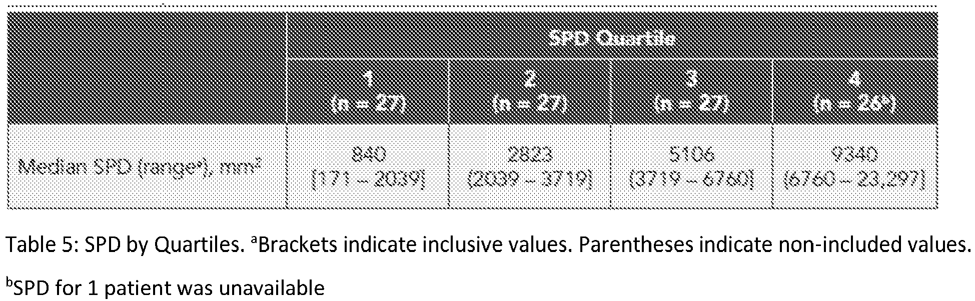

The rate of ongoing response decreases with higher quartiles of SPD (FIG. 1). Also, grade ≥ 3 CRS and neurologic events occurred least frequently in patients within the lowest quartile of SPD.

The results demonstrated that axi-cel showed long term clinical benefit for patients with refractory large B cell lymphoma, regardless of the number of prior lines of therapy and SPD. Also, axi-cel was manufactured with similar product characteristics across all lines of therapy. CAR peak expansion, area under the curve, and ranges are comparable across patients with 1-4 prior lines of therapy. In additon, the study showed that higher rates of ongoing responses at 1 year were observed in patients with lower SPD, and that lower rates of CRS and neurologic events were observed in patients whose index lesion SPD was within the lowest quartile of SPD.

### Example 2

### Long-term Activity of Axicabtagene Ciloleucel in Refractory Large B-cell Lymphoma

In this study, more than 100 patients with refractory large B-cell lymphoma were treated with axicabtagene ciloleucel and their cancer progression followed by more than two years after a single treatment. The results show that axicabtagene ciloleucel may induce durable responses and a median overall survival of greater than 2 years. The outcomes were similar across all patient subgroups, which included a large proportion of patients with activated B-cell-like, double expressor, and high-grade B-cell lymphoma.

119 patients were enrolled and 108 received treatment. The patient distribution across the number of lines of prior therapy was as follows: Phase 1: Median (IQR) 3 (3-4); 1 line (0 patients); 2 lines (1 patient, 14%), 3 lines (6 patients, 86%). Phase 2: Median (IQR) 3 (2-4); 1 line (3 patients, 3%); 2 lines (28 patient, 28%), 3 lines (70 patients, 69%). In addition, in Phase 1, 1 patient (14%) had an history of primary refractory disease and 1 patient (14%) had an history of resistance to two consecutive lines. In Phase 2, 26 patients (26%) had an history of primary refractory disease and 54 patients (53%) had an history of resistance to two consecutive lines. Patients could have had other therapies after primary refractory disease.

With regard to cancer characterization, 52 (70%) of 74 patients assessed for cell of origin had germinal centre B-cell-like disease and 18 (24%) had activated B-cell-like disease. Of the 47 patients with pretreatment tumour samples, 30 (64%) had double expressor B-cell lymphoma and seven (15%) had high-grade B-cell lymphoma, including one (2%) with triple-hit high-grade B-cell lymphoma, four (9%) with double-hit high-grade B-cell lymphoma, and two (4%) with high-grade B-cell lymphoma not otherwise specified.

101 patients assessable for activity in phase 2 were followed up for a median of 27.1 months (IQR 25.7-28.8). According to investigator assessment, 84 (83%) of 101 patients had an objective response to the treatment, 59 (58%) complete responses, and 25 (25%) partial responses. Ten (10%) patients had stable disease, five (5%) had progressive disease as best response, and two (2%) could not be assessed.

Median time to response was 1 month (IQR 1-1). 11 of 33 patients with partial responses at 1 month, and 11 of 24 patients with stable disease at 1 month, subsequently achieved a complete response, with many conversions occurring by 6 months. Among the 33 patients with double-expressor and high-grade B-cell lymphoma, 30 (91%) exhibited an objective response and 23 (70%) exhibited a complete response. The median duration of response for all 101 patients was 11.1 months (95% C! 4.2-not estimable).

Ongoing responses were consistent across key baseline and clinical covariates. Median progression free survival was 5.9 months (95% C! 3.3-15). The estimated proportion of patients with progression-free survival at 24 months was 72% (95% Cl 56.0-83.0) among those with complete responses at 3 months, 75% (31.5-93.1) among those with partial responses at 3 months, and 22.2% (3.4-51.3) among those with stable disease at 3 months. The median overall survival was not reached (95% C! 12.8-not estimable), with an estimated 24-month survival proportion of 50.5% (95% C! 40.2-59.7). No patients were lost to follow-up.

Ongoing response at 24 months was associated with higher CAR T-cell peak concentrations and area under the curve in the first 28 days after infusion. By 24 months, 11 (34%) of 32 assessable patients maintained ongoing responses, no longer had detectable engineered T cells. The safety profile of 2 years after infusion was similar to those of shorter timeline.

Analysis of progression free survival by response at 3 months suggests that achievement of complete or partial responses at 3 months might be predictive of long-term response durability (FIG. 2). Three months after infusion, six (17%) of 35 assessable patients with ongoing responses had detectable B cells in peripheral blood. At 9 months, 20 (61%) of 33 assessable patients had detectable B cells, and, at 24 months, 24 (75%) of 32 assessable patients had detectable B cells.

These results suggest that patients with refractory large B-cell lymphoma treated with a single infusion of axicabtagene ciloleucel exhibited durable responses lasting more than 2 years and needed no further consolidation therapy. In this population of patients refractory to several lines of treatment, which included a large proportion of patents with activated B-cell-like, double expressor, and high-grade B-cell lymphoma, outcomes were similar across all patient subgroups. Median overall survival was not reached at 2 years, with an estimated 24-month survival proportion of 50.5% (95% C! 40.2-59.7). In contrast, the expected median overall survival with conventional therapies is approximately 6 months, with a 2-year overall survival of approximately 20%.

Despite targeting of CD19 and the expected induction of B-cell aplasia, the frequency of late-onset grade 3 or worse serious infections was low. 75% of assessable patients with ongoing responses showed evidence of B-cell recovery by 24 months, and initiation of B-cell recovery was noted in some patients at 9 months. These patients with ongoing responses recovered B cells suggests the possibility that durable responses in adults with lymphoma may not require long term persistence of functional CART cells.

### Example 3

### Efficacy and Safety Outcomes of Patients ≥ 65 Years of Age in a Phase 1/2 Study of Axicabtagene Ciloleucel (Axi-Cel) in Refractory Large B Cell Lymphoma (LBCL)

Eligible patients with refractory large B-cell lymphoma (LBCL) underwent leukapheresis and received cyclophosphamide 500 mg/m² IV and fludarabine 30 mg/m² IV, both given on the fifth, fourth, and third day before receiving axicabtagene ciloleucel at a dose of 2 × 10⁶ CAR-positive viable T cells/kg via IV infusion (Day 0). 108 patients were treated. Patients ≥ 65 y (n = 27) vs < 65 y (n = 81) had a median age of 69 years vs 55 years (y), respectively, were 81% vs 63% male; 70% vs 36% had an IPI score 3-4; 59% vs 57% had ECOG 1; 81% vs 84% were at a disease stage III/IV; 67% vs 72% had ≥ 3 prior therapies; and median tumor burdens by SPD (range) were 3790 (600-16764) mm² vs 3574 (171 - 23297) mm². CAR T cell expansion by peak level (43 vs 35 cells/µl) or area under the curve (562 vs 448 d × cells/µl) was similar in patients ≥ 65 years (y) vs < 65 y, respectively. With regard to disease type, 74% vs 79% had DLBCL; 0% vs 10% had PMBCL; and 26% vs 11% had TFL. 19% vs 30% had prior ASCT. The refractory subgroups before enrollment were distributed as follows: 4% vs 2% were primary refractory; 78% vs 73% were refractory to second- or later line therapy; and 19% vs 25% were in relapse post-ASCT.

Median follow-up was 27.1 months for Phase 2 patients (n = 101). The efficacy by age group was distributed as follows: the ORR for patients ≥ 65 y (n = 24) and < 65 y (n = 77) was 92% and 81% (CR rate 75% and 53%; PR 17% vs 27%), respectively, with ongoing responses in 42% and 38% of patients (ongoing CR 42% and 35%). The 24-mo OS rate was 54% for patients ≥ 65 y and 49% for patients < 65 y.Grade ≥ 3 AEs were observed in100% of patients ≥ 65 y and 98% of patients < 65 y). Grade ≥ 3 neurologic events and cytokine release syndrome occurred in 44% vs 28% and 7% vs 12% of patients > 65 y vs < 65 y, respectively. Rates of Grade ≥ 3 cytokine release syndrome (CRS) and neurologic events (NE) were similar across age groups. CRS includes pyrexia, hypotension, and hypoxia. NEs include encephalopathy, confusional state, aphasia, agitation, and delirium. Any-grade and Grade ≥ 3 cytopenias (cytopenias, thrombocytopenia, neutropenia, anemia) were consistent across age groups. 26% and 32% of patients ≥ 65 and < 65 years of age, respectively, received intravenous immunoglobulin therapy.

Compared to patients <65 years, patients ≥ 65 vs exhibited disease progression as best response to last prior therapy and an IPI score of 3 to 4 (age ≥ 60 years being a component of IPI scoring). Axi-cel may induce high rates of durable responses with a manageable safety profile for patients ≥ and < 65 years. Older patients with refractory LBCL generally have limited treatment options. The results of 2-year analysis illustrated treatment efficacy (83% objective response rate; 58% complete response rate; 39% ongoing responses; N = 101; median follow-up, 27.1 months) and safety (late-onset adverse events were primarily manageable infections, and there were no late-onset axi-cel-related incidences of cytokine release syndrome, neurologic events, or deaths; N=108).
In particular, the present invention pertains to the following:
1. A method of predicting a response to CD19 CAR-T treatment in a subject having cancer, comprising measuring a baseline SPD in the subject, determining the SPD range, wherein a low SPD range indicates a likelihood of positive response to the CAR-T treatment.
2. The method of item 1, where the SPD can fall within one of 4 ranges, whereby the lower the range in which the SPD falls the higher the likelihood of response to CD19 CAR-T treatment is.
3. The method of item 2, wherein the four ranges comprise the following:
   SPD Quartile 1, from about 100 (inclusive) to about 2000 mm² (inclusive), median SPD of about 840;
   SPD Quartile 2, from about 2000 (non inclusive) to about 3700 mm² (inclusive), median SPD of about 2820;
   SPD Quartile 3, from about 3700 (non inclusive) to about 6700 mm² (inclusive), median SPD of about 5100; and
   SPD Quartile 4, from about 6700 (non inclusive) to about 24,000 mm² (inclusive), median SPD of about 9300.
4. The method of any one of items 1 through 3, wherein the subject is subsequently treated with CD19 CAR-T treatment when the baseline SPD value is in the SPD Quartiles 1 through 4.
5. A method of treating cancer in a subject in need thereof, comprising administering a therapeutically effective amount of a CD19 CAR-T treatment to a subject in which the baseline SPD value is in the SPD Quartiles 1 through 4.
6. A method of predicting a likelihood of relapse after CD19 CAR-T treatment in a subject having cancer, comprising determining the number of lines of prior therapy in the subject, determining where the number falls within one of four ranges, whereby the higher the number of lines of prior therapy the higher likelihood of relapse after CD19 CAR-T treatment for the subject is predicted to be.
7. A method of predicting the likelihood of ongoing response to CD19 CAR-T treatment in a subject having cancer, comprising measuring the baseline number of lines of prior therapy in the subject, determining where the number falls within one of four ranges, whereby the lower the range indicates the likelihood of ongoing response to CD19 CAR-T treatment.
8. The method of any one of items 6 and 7, wherein the ranges of number of lines of prior therapy are 1-2; 3; 4; or ≥ 5.
9. The method of any one of items 6 through 8, further comprising subsequently administering CD19 CAR-T treatment to the subject in which the number of lines of prior therapy are 1-2; 3; 4; or ≥ 5.
10. A method of treating cancer in a subject in need thereof, comprising administering a therapeutically effective amount of a CD19 CAR-T treatment to a subject in which the number of lines of prior therapy in the subject is 1-2; 3; 4; or ≥ 5.
11. The method of any one of items 1-10, wherein the cancer is a hematologic cancer or relapsed/refractory diffuse large B cell lymphoma.
12. The method of any one of items 1-11, wherein the CD19 CAR-T treatment comprises treatment with axicabtagene ciloleucel (Yescarta), tisagenlecleucel (Kymriah), JCAR017, JCAR015, JCAR014, Uppsala U. anti-CD19 CAR (NCT02132624), or UCART19.
13. A method of predicting long-term response durability to treatment of cancer with anti-CD19 CAR-T cell treatment in a patient in need thereof, the method comprising assessing progression free survival at 3 months after a single dose of treatment, wherein achievement of complete or partial response at 3 months is predictive of long-term response durability in the patient.
14. The method of item 13, wherein the anti-CD19 CAR-T treatment comprises treatment with axicabtagene ciloleucel (Yescarta), tisagenlecleucel (Kymriah), JCAR017, JCAR015, JCAR014, Uppsala U. anti-CD19 CAR (NCT02132624), or UCART19.
15. The method of item 13 or 14, wherein the cancer is a hematological cancer.
16. The method of item 15, wherein the cancer is relapsed/refractory diffuse large B cell lymphoma.
17. The method of any one of items 13 through 16, wherein long-term response durability comprises a complete or partial response lasting more than 9 months, more than 12 months, more than 18 months, or more than 24 months.
18. The method of any one of items 1 through 17, wherein the subject is ≥ 65 years old.
19. The method of any one of items 1 through 17, wherein the subject is < 65 years old.
20. The method of any one of items 1 through 19, whrein the CD19 CAR-T treatment is administered as first line therapy.

## Claims

1. A method of predicting a likelihood of relapse after anti-CD19 chimeric antigen receptor (CAR)-T cell treatment in a subject having cancer that expresses CD19, comprising determining the number of lines of prior therapy in the subject, determining where the number falls within one of four ranges, whereby the higher the number of lines of prior therapy the higher likelihood of relapse after anti-CD 19 CAR-T cell treatment for the subject is predicted to be, wherein the ranges of number of lines of prior therapy are 1-2; 3; 4; or ≥ 5.

2. Anti-CD 19 chimeric antigen receptor (CAR)-T cells for use in a method of treating cancer in a subject in need thereof, said method comprising the steps of claim 1 and further comprising administering a therapeutically effective amount of anti-CD 19 CAR-T cells to the subject in which the number of lines of prior therapy in the subject is 1-2; 3; 4; or ≥ 5.

3. The method of claim 1 or the anti-CD 19 CAR-T cells for use of claim 2, wherein the cancer is a hematologic cancer or is relapsed/refractory diffuse large B cell lymphoma.

4. The method of claim 1 or 3 or the anti-CD 19 CAR-T cells for use of claim 2 or 3, wherein the anti-CD19 CAR-T cells administered are selected from axicabtagene ciloleucel (Yescarta), tisagenlecleucel (Kymriah), JCAR017, JCAR015, JCAR014, Uppsala U. anti-CD19 CAR (NCT02132624), or UCART19.

5. The method of claim 1, 3 or 4 or the anti-CD 19 CAR-T cells for use of any one of claims 2 to 4, wherein the subject is ≥ 65 years old.

6. The method of claim 1, 3, or 4 or the anti-CD 19 CAR-T cells for use of any one of claims 2 to 4, wherein the subject is < 65 years old.

7. The method of any one of claims 1 and 3 to 6 or the anti-CD 19 CAR-T cells for use of any one of claims 2 to 6, wherein the anti-CD 19 CAR-T cells are administered as first line therapy.

8. The method of any one of claims 1 and 3 to 6 or the anti-CD 19 CAR-T cells for use of any one of claims 2 to 6, wherein the subject has received at least one line of prior therapy.
